(19) Europäisches Patentamt
European Patent Office
Office européen des brevets

(11) EP 3 322 305 B1

(12) EUROPEAN PATENT SPECIFICATION

(45) Date of publication and mention of the grant of the patent:
**01.07.2020 Bulletin 2020/27**

(21) Application number: **16736070.0**

(22) Date of filing: **05.07.2016**

(51) Int Cl.:
*C12N 9/78* (2006.01) *C12N 9/80* (2006.01)
*A23J 3/14* (2006.01) *A23L 33/185* (2016.01)
*A23P 30/40* (2016.01) *A23L 29/206* (2016.01)

(86) International application number:
**PCT/EP2016/065791**

(87) International publication number:
**WO 2017/009100 (19.01.2017 Gazette 2017/03)**

(54) **USE OF PEPTIDYLARGININE DEIMINASE TO SOLUBILIZE PROTEINS, OPTIONALLY FURTHER TO REDUCE THEIR FOAMING TENDENCY**

VERWENDUNG VON PEPTIDYLARGININ-DEIMINASE ZUR LÖSUNG VON PROTEINEN, GEGEBENENFALLS WEITERS ZUR REDUZIERUNG IHRER SCHAUMNEIGUNG

UTILISATION DE PEPTIDYLARGININE-DÉSIMINASE POUR SOLUBILISER DES PROTÉINES, ÉVENTUELLEMENT POUR RÉDUIRE ENCORE LEUR TENDANCE MOUSSANTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.07.2015 EP 15176470**

(43) Date of publication of application:
**23.05.2018 Bulletin 2018/21**

(73) Proprietor: **DSM IP Assets B.V.**
**6411 TE Heerlen (NL)**

(72) Inventors:
- **VLASIE, Monica Diana**
  **6100 AA Echt (NL)**
- **VEERMAN, Cecile**
  **6100 AA Echt (NL)**

(74) Representative: **DSM Intellectual Property**
**P.O. Box 4**
**6100 AA Echt (NL)**

(56) References cited:
**WO-A1-95/28850** **WO-A1-2008/000714**
**WO-A2-2011/100369**

- **TOMOFUMI KUROKAWA ET AL: "Conversion of Peanut Trypsin-Chymotrypsin Inhibitor B-V to a Chymotrypsin Inhibitor by Deimination of the P1 Arginine Residues in Two Reactive Sites1", J. BIOCHEM, vol. 101, no. 101, June 1987 (1987-06), pages 1361-1367, XP055308056,**
- **YONG YIE HUI ET AL: "Effects of enzymatic deamidation by protein-glutaminase on structure and functional properties of wheat gluten", JOURNAL OF AGRICULTURAL AND FOOD CHEMISTRY, AMERICAN CHEMICAL SOCIETY, US, vol. 54, no. 16, 9 August 2006 (2006-08-09), pages 6034-6040, XP002626965, ISSN: 0021-8561, DOI: 10.1021/JF060344U [retrieved on 2006-07-19]**
- **E. TARCSA ET AL: "The Fate of Trichohyalin: SEQUENTIAL POST-TRANSLATIONAL MODIFICATIONS BY PEPTIDYL-ARGININE DEIMINASE AND TRANSGLUTAMINASES", JOURNAL OF BIOLOGICAL CHEMISTRY, vol. 272, no. 44, 31 October 1997 (1997-10-31), pages 27893-27901, XP055246680, US ISSN: 0021-9258, DOI: 10.1074/jbc.272.44.27893**
- **AZUMA N ET AL: "ENZYMIC MODIFICATION OF ALPHA-S-1 CASEIN WITH PEPTIDYLARGININE DEIMINASE PREPARATION OF LESS ACID-COAGULABLE AND LESS CALCIUM-SENSITIVE CASEIN", JOURNAL OF DAIRY RESEARCH, CAMBRIDGE UNIVERSITY PRESS, CAMBRIDGE, GB, vol. 58, no. 4, 1991, pages 421-429, XP009188300, ISSN: 0022-0299**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 322 305 B1

- **NORIHIRO AZUMA ET AL: "Role of the Arginyl Residues of x-Casein in Micelle Formation Effect of Deimination on ~sl-~-Casein Complex Formation", INT. DAIRY JOURNAL, vol. 4, no. 3, January 1994 (1994-01), pages 193-204, XP055246736,**

SEQUENCE LISTING

<110> DSM IP Assets B.V.

<120> USE OF ENZYME TO IMPROVE A PHYSICAL PROPERTY OF A PROTEIN

<130> 31264-WO-PCT

<160> 3

<170> BiSSAP 1.2

<210> 1
<211> 640
<212> PRT
<213> Fusarium graminearum

<400> 1

Met His Leu Leu Asn Gly Lys Thr Ala Ala Val Ala Leu Ala Leu Leu
1               5                   10                  15
Asn Ser Cys Asn Ala Leu Lys Val Thr Ile Leu Ala Asp Thr Asn Arg
            20                  25                  30
Asp Gly Lys Val Asp Asn Asn Asp Ile Asn Gly Lys Ser Thr Trp Thr
        35                  40                  45
Asn Asn Arg Gly Ala Leu Ile Leu Pro Asn Ile Gly Asp Thr Gly Ser
    50                  55                  60
Arg Cys Ala Lys Gln Trp Gly Pro Ser Val Asp Ile Gln Gly Asp Glu
65                  70                  75                  80
Ser Tyr Leu Asp Lys Cys Asn Asp Ala Ser Asp Asn Val Gln Arg Asn
                85                  90                  95
Pro Lys Tyr Leu Ala Ser Leu Lys Thr Leu Pro Leu Thr Thr Leu Ser
            100                 105                 110
Ala Thr Ala Lys Gly Ser Ile Ile Ile Ala Asp Lys Thr Gly Ala Ser
        115                 120                 125
Lys Val Arg Ile Phe Val Lys Gln Ser Gly Lys Trp Asn Tyr Val Ala
    130                 135                 140
Ala Asp His Val Phe Thr Ala Lys Glu Leu Lys Ser Gly Leu Glu Leu
145                 150                 155                 160
Gly Val Asp Ala Arg Asp Val Arg Arg Pro Gln Asp Trp Asn Gly Tyr
                165                 170                 175
Ala Lys Ile Gln Phe Thr Val Thr Asp Gly Lys Thr Lys Ala Thr Asp
            180                 185                 190
Ala Val Ala Val Arg Val Ala Pro Val Leu Thr His His His Gly Gln
        195                 200                 205
His Ala Gln Arg Ile Phe Thr Thr Gly Val Asn Glu Ala Gly Val Asn
    210                 215                 220
Lys Val Gln Glu Thr Phe Ile Ala Asp Ile Leu Arg Asn Val Ala Gly
225                 230                 235                 240
Ala Gly Ile Lys Glu Pro Val Phe Gln Phe His Asn Gln Asp Ile Trp
                245                 250                 255
Thr Gln Asp Phe Phe Glu Pro Gly Tyr Ala Ser Ile Pro Gly Pro Asn
            260                 265                 270
Gly Pro Val Ser Ile Arg Ile Met Ile Arg Ser Ala Gln Ser Ser Arg
        275                 280                 285
Arg Ser Gly Arg Asp Ala Phe His Asp Leu Arg Asn Asp Gln Val Gly
    290                 295                 300
Ala Val Gln His Pro Gly Asp Gly Asp Ser Ile Asp Ser Thr Gly Asn
305                 310                 315                 320
Leu Glu Thr Ile Pro Pro Tyr Ser His Asn Gly Lys Ser Phe Pro Val
                325                 330                 335
Gly Arg Thr Ile Met Gly Ala Trp Asp Gly Arg Ala Pro Leu Met Val
            340                 345                 350

```
Glu Phe Leu Lys Ala Gln Gln Val Gln Glu Pro Leu Ile Leu Asp Thr
        355                 360                 365
Ser Trp Leu Tyr Val Gly His Val Asp Glu Phe Ile Gln Phe Leu Pro
    370                 375                 380
Ser Asn Asn Lys Leu Gly Trp Val Ile Met Val Ala Asp Pro Met Lys
385                 390                 395                 400
Gly Val Asp Leu Leu Lys Lys Ala Val Lys Thr Gly His Gly Lys Val
                405                 410                 415
Lys Ala Val Ser Arg Pro Leu Ser Ala Asp Glu Lys Lys Glu Gln Leu
            420                 425                 430
Cys Leu Pro Arg Gln Thr Ile Ala Glu Ala Leu Lys Phe Lys Ser Phe
        435                 440                 445
Asp Ala Ile Asn Lys His Ser Ala Glu Arg Ile Gln Ala Asn Leu Asp
    450                 455                 460
Ile Ile Lys Arg Glu Thr Gly Ile Thr Asp Glu Asp Ile His Arg Val
465                 470                 475                 480
Pro Ala Leu Phe Tyr Tyr Thr Gln Ser Asn Ser Trp Leu Cys Pro Gly
                485                 490                 495
Glu Thr Ala Glu Asp Asp Ser Ala Gln Pro Gln Lys Ala Ala Ser Asn
            500                 505                 510
Ser Gly Ile Thr Met Lys Thr Ser Gln Gly Gly Pro Gly Phe Lys Ala
        515                 520                 525
Lys Ser Ile Val Glu Ala Ala Thr Pro Gly Lys Ser Ile Gln Arg Arg
    530                 535                 540
Val Ile Asp Pro Ala Thr Gln Val Thr Ala Leu Tyr Pro Gly Ser Val
545                 550                 555                 560
Asn Gly Leu Val Met Thr Asp Thr Lys Ile Leu Ala Pro Ser Pro Trp
                565                 570                 575
Gly Pro Val Ile Asn Lys Gln Asp Ile Phe Ala Ala Ala Val Ser Gln
            580                 585                 590
Val Tyr Thr Asn Ala Gly Tyr Asn Val Thr Tyr Gln Asp Asp Trp Phe
        595                 600                 605
Ser His Phe Lys Leu Gln Gly Asp Val His Cys Gly Ser Asn Ser Trp
    610                 615                 620
Arg Glu Ile Pro Lys Lys Trp Trp Asp Ser Leu Arg Val Asn Asn Tyr
625                 630                 635                 640
```

<210> 2
<211> 320
<212> PRT
<213> Chryseobacterium proteolyticum

<220>
<223> strain 9670

<400> 2

```
Met Lys Asn Leu Phe Leu Ser Met Met Ala Phe Val Thr Val Leu Thr
1               5                   10                  15
Phe Asn Ser Cys Ala Asp Ser Asn Gly Asn Gln Glu Ile Asn Gly Lys
            20                  25                  30
Glu Lys Leu Ser Val Asn Asp Ser Lys Leu Lys Asp Phe Gly Lys Thr
        35                  40                  45
Val Pro Val Gly Ile Asp Glu Glu Asn Gly Met Ile Lys Val Ser Phe
    50                  55                  60
Met Leu Thr Ala Gln Phe Tyr Glu Ile Lys Pro Thr Lys Glu Asn Glu
65                  70                  75                  80
Gln Tyr Ile Gly Met Leu Arg Gln Ala Val Lys Asn Glu Ser Pro Val
                85                  90                  95
His Ile Phe Leu Lys Pro Asn Ser Asn Glu Ile Gly Lys Val Glu Ser
            100                 105                 110
Ala Ser Pro Glu Asp Val Arg Tyr Phe Lys Thr Ile Leu Thr Lys Glu
        115                 120                 125
Val Lys Gly Gln Thr Asn Lys Leu Ala Ser Val Ile Pro Asp Val Ala
    130                 135                 140
Thr Leu Asn Ser Leu Phe Asn Gln Ile Lys Asn Gln Ser Cys Gly Thr
145                 150                 155                 160
Ser Thr Ala Ser Ser Pro Cys Ile Thr Phe Arg Tyr Pro Val Asp Gly
                165                 170                 175
Cys Tyr Ala Arg Ala His Lys Met Arg Gln Ile Leu Met Asn Asn Gly
            180                 185                 190
Tyr Asp Cys Glu Lys Gln Phe Val Tyr Gly Asn Leu Lys Ala Ser Thr
        195                 200                 205
Gly Thr Cys Cys Val Ala Trp Ser Tyr His Val Ala Ile Leu Val Ser
    210                 215                 220
Tyr Lys Asn Ala Ser Gly Val Thr Glu Lys Arg Ile Ile Asp Pro Ser
225                 230                 235                 240
Leu Phe Ser Ser Gly Pro Val Thr Asp Thr Ala Trp Arg Asn Ala Cys
                245                 250                 255
Val Asn Thr Ser Cys Gly Ser Ala Ser Val Ser Ser Tyr Ala Asn Thr
                260                 265                 270
Ala Gly Asn Val Tyr Tyr Arg Ser Pro Ser Asn Ser Tyr Leu Tyr Asp
        275                 280                 285
Asn Asn Leu Ile Asn Thr Asn Cys Val Leu Thr Lys Phe Ser Leu Leu
    290                 295                 300
Ser Gly Cys Ser Pro Ser Pro Ala Pro Asp Val Ser Ser Cys Gly Phe
305                 310                 315                 320
```

<210> 3
<211> 1000
<212> DNA
<213> Artificial Sequence

<220>
<221> source
<222> 1..1000
<223> /organism="Artificial Sequence" /note="Nucleotide sequence encoding protein glutaminase from Chryseobacterium proteolyticum strain 9670 with a PacI restriction site and ribosome binding site at the 5\222- end, and a double stop codon and PmeI restriction site at the 3\222 - end" /mol_type="unassigned DNA"

<400> 3

```
ttaattaaaa aaggagcgat ttacatatga aaaatctttt tttatcaatg atggcctttg      60
tgaccgtctt aacttttaat tcctgtgccg attccaacgg gaatcaggaa atcaacggaa     120
aggaaaaact aagtgtaaat gattctaagc tgaaagattt cggaaagact gtaccggtag     180
ggatagacga agaaaacgga atgataaagg tgtcatttat gttaactgcg caattctatg     240
aaattaagcc gaccaaagaa aatgagcagt atatcggaat gcttagacag gctgttaaga     300
atgaatctcc tgtacacatt ttcttaaagc ctaatagcaa tgaaatagga aaagtggagt     360
ctgcaagtcc ggaagacgta agatatttta aaacgatcct gacaaaagaa gtaaaagggc     420
aaaccaataa attggcgagt gtaattcctg atgtagctac attaaattct ttattcaatc     480
aaataaagaa tcagtcttgc ggtacctcta cggcgtcctc accatgcatc acattcagat     540
atcctgtaga cggatgttat gcaagagccc ataagatgag acaaatctta atgaacaacg     600
```

-

```
gctatgactg tgaaaaacaa tttgtatacg gaaacctaaa ggcatcaaca ggaacttgct     660
gtgtggcgtg gagctaccac gttgcaatat tggtaagcta taaaaatgct tccggagtaa     720
cggaaaaaag aattattgat ccttcactat tttcaagcgg tcctgtaaca gatacagcat     780
ggagaaacgc ttgcgttaac acctcttgcg gatctgcatc cgtttcctct tatgctaata     840
ctgcaggaaa tgtttattac agaagtccta gtaattctta cctgtatgac aacaatctga     900
tcaataccaa ctgtgtactg actaaatttt cactgctttc cggatgttct ccttcacctg     960
caccggatgt atccagctgt ggattttaat aagtttaaac                          1000
```

## Description

The present invention relates to the use of an enzyme to improve the solubility of proteins.

## Background

In a world with a growing population there is an increased demand of proteins. To respond to this growing demand of proteins, there is a need to look at wider applications of proteins. In addition it is desired to look for plant proteins as an alternative for animal proteins, since it is considered that plants are a more sustainable source of proteins than animals. The use of plant proteins in food is still limited because the solubility is usually low, which is an indication poor functional properties. Solubility of the proteins can be improved by proteolysis. However this can lead to destruction of other functional properties, such as gelling, emulsification or other sensory attributes. Yong et al, J. of Agric. Food Chem. 2006, 54, 6034-6040 discloses the use of protein glutaminase to increase the solubility of wheat gluten. Protein glutaminase deamidates proteins and thereby increase the charge of the deamidated protein, which improves the solubility of the protein, without proteolysis of the protein.

There is a need for alternative enzymatic ways to improve the solubility of proteins.

## Detailed description

The present invention relates to the use of peptidyl arginine deiminase to improve solubility of a plant protein at a pH of between 5 and 8.5, wherein the plant protein is pea, soy or cereal. Other physical properties of proteins include gelling, foaming or emulsification properties.

In one embodiment the present disclosure relates to the use of peptidyl arginine deiminase to improve solubility of a plant protein at a pH of between 5 and 8.5, wherein the plant protein is pea, soy or cereal.

The present disclosure further relates to a process to improve solubility of a plant protein comprising incubating the plant protein with a peptidyl arginine deiminase at a pH of between 4 and 9, wherein the plant protein shows improved solubility at a pH of between 5 and 8.5, wherein the plant protein is pea, soy or cereal protein.

To improve a physical property of a protein in a use or process as disclosed herein includes to solubilize the protein and optionally to reduce foam capacity of the protein.

In one embodiment the present invention relates to the use of peptidyl arginine deiminase to solubilize plant proteins selected from pea, soy or cereal at a pH of between 5 and 8,5. To solubilize as used herein comprises incubating the plant protein with a peptidyl arginine deiminase at a pH of between 4 and 9, such as a pH of between 5 and 8, or a pH of between 5,5 to 7,5 or a pH of between 6 and 7 or a pH of between 6.2 and 6.8, or pH of 6.5 and solubilizing protein in a solution having a pH of between 5 and 8,5.

Peptidyl arginine deiminases are for instance known from WO2008/000174. WO2008/000174 discloses a process for enzymatically treating a protein with a protein arginine deiminase, wherein at least 30% of the arginine is transformed into citrulline. We surprisingly found that when protein arginine deiminase is incubated with proteins selected from pea, soy or cereal the solubility of the proteins is increased, for instance the solubility of protein is increased at a neutral pH, as compared to a protein that has not been incubated with a protein arginine deiminase, without influencing other functional properties of the protein, or proteolysis of the protein.

The term protein arginine deiminase and peptidyl arginine deiminase (PAD) are used interchangeably herein. Protein or peptidyl arginine deiminases belong to a family of enzymes (EC 3.5.3.15) which convert peptide or protein bound arginine into peptide or protein bound citrulline. This process is called deamination or citrullination. In the reaction from arginine to citrulline, one of the terminal nitrogen atoms of the arginine side chain is replaced by an oxygen. The reaction uses one water molecule and yields ammonia as a side product (http://en.wikipedia.org/wiki/Citrullination). Whereas arginine is positively charged at a neutral pH, citrulline is uncharged. Surprisingly, it was found that a protein wherein at least part of the arginine has been converted into citrulline, and thereby resulting in protein with less charge, exhibited an increased solubility, in a solution having a pH of between 5 and 8.5, or a pH of between 5.5 and 8.

Solubility of a protein as used herein is the amount of nitrogen in the supernatant after solid-liquid separation of the protein. The amount of nitrogen may be measured after incubation of the protein with PAD and / or incubation of the protein without PAD. Separation of the protein may be performed by centrifugation or filtration. The nitrogen content can be measured by the Kjeldahl method. Solubilizing protein is measured as solubilizing nitrogen.

In one embodiment, the solubility of protein is increased by the use of peptidyl arginine deiminase. The increase in solubility of protein is at least 2%, 3%, 5%, 10%, for instance at least 20%, 25%, 30%, 35%, 40%, 45%, 50%, 55%, 60%, 65%, 70%, 75%, 80%, 85% or at least 90% or 95%. The increase in solubility of a protein that has been incubated with PAD is compared to a protein that has not been incubated with PAD under the same conditions.

Surprisingly, it was also found that the foam capacity of a protein as used herein that had been treated with a peptidyl arginine deiminase was reduced, for instance the foam capacity was reduced with at least 2%, 3%, 4%, 5% or 6%. A reduced foam capacity is advantageous in particular in a beverage comprising a plant protein, for instance milk derived from a plant source.

Foam capacity as used herein is determined by dividing the foam volume immediately after mixing a beverage by the volume of the liquid before mixing multiplied by 100.

Plant proteins selected from pea, soy or cereal may be used in a use or process of the present invention. Advantageously a protein comprises protein bound arginine, such as at least 1 mol%, 2, 3, 4, 5 or at least 6 mol. Examples of plant protein are cereal protein, potato protein, soy protein, rapeseed protein, rice protein, pea protein as well as proteins from other plants known to be rich in arginine such as lupins, sesame, palm pits etc. According to the invention the plant protein is pea, soy or cereal protein. Examples of cereal protein are wheat or maize or fractions thereof for example, gluten such as wheat gluten.

In one embodiment, use of peptidyl arginine deiminase to improve solubility and optionally to reduce foam capacity, comprises incubating a plant protein chosen from pea, soy or cereal with a peptidyl arginine deiminase at a suitable temperature and pH, for instance incubating protein with a peptidyl arginine deiminase at a pH of between 4 and 9, such as a pH of between 5 and 8.5, such as a pH of between 5.5 and 8, such as a pH between 6 and 7, or a pH of between 6.2 and 6.8, for instance at a pH of about 6.5. A suitable temperature at which protein is incubated with PAD may be between 20 and 60 degrees Celsius, such as a between 30 and 50, or between 35 and 45 degrees Celsius

In one embodiment, a physical property of protein is improved, when measuring the physical property of the protein in a solution having a pH of between 5 and 8.5, such as a pH of between 5.5 and 8, such as a pH between 6 and 7, or a pH of between 6.2 and 6.8.

Peptidyl arginine deiminase (PAD) may be derived from any suitable origin, for instance from mammalian or microbial origin. PAD's used in the present invention are advantageously derived from a microbial source. For instance, PAD's may be derived from fungal origin such as from *Fusarium sp.* such as *Fusarium graminearum, Chaetomium globosum, Phaesphaeria nodorum* or from bacterial origin such as from the bacteria *Streptomyces, eg Streptomyces scabies, Streptomyces clavuligeres.* The wording "derived" or "derivable" from with respect to the origin of a polypeptide as disclosed herein, means that when carrying out a BLAST search with a polypeptide as disclosed herein, the polypeptide may be derivable from a natural source, such as a microbial cell, of which an endogenous polypeptide shows the highest percentage homology or identity with the polypeptide as disclosed herein.

A peptidyl arginine deiminase may be a pure or purified peptidyl arginine deiminase. A pure of purified peptidyl arginine deiminase is an enzyme that may be at least 50% pure, e.g., at least 60% pure, at least 70% pure, at least 75% pure, at least 80% pure, at least 85% pure, at least 80% pure, at least 90% pure, or at least 95% pure, 96%, 97%, 98%, 99%, 99.5%, 99.9% pure for instance as determined by SDS-PAGE or any other analytical method suitable for this purpose and known to the person skilled in the art.

Advantageously, peptidyl arginine deiminase is a polypeptide which has at least 80%, 85%, 90%, 95%, 96%, 97%, 98%, 99% or 100% identity to the amino acid sequence of SEQ ID NO: 1, or to the mature amino acid sequence of SEQ ID NO: 1, wherein the polypeptide has peptidyl arginine deiminase activity.

For the purpose of this invention, it is defined here that in order to determine the percentage of sequence identity of two amino acid sequences, the sequences are aligned for optimal comparison purposes. In order to optimize the alignment between the two sequences gaps may be introduced in any of the two sequences that are compared. Such alignment can be carried out over the full length of the sequences being compared. Alternatively, the alignment may be carried out over a shorter length, for example over about 20, about 50, about 100 or more amino acids. The sequence identity is the percentage of identical matches between the two sequences over the reported aligned region. The percent sequence identity between two amino acid sequences may be determined using the Needleman and Wunsch algorithm for the alignment of two sequences. (Needleman, S. B. and Wunsch, C. D. (1970) J. Mol. Biol. 48, 443-453). Both amino acid sequences and nucleotide sequences can be aligned by the algorithm. The Needleman-Wunsch algorithm has been implemented in the computer program NEEDLE. For the purpose of this invention the NEEDLE program from the EMBOSS package was used (version 2.8.0 or higher, EMBOSS: The European Molecular Biology Open Software Suite (2000) Rice,P. Longden, I. and Bleasby, A. Trends in Genetics 16, (6) pp276-277, http://emboss.bioinformatics.nl/). For protein sequences EBLOSUM62 is used for the substitution matrix. The optional parameters used are a gap-open penalty of 10 and a gap extension penalty of 0.5. The skilled person will appreciate that all these different parameters will yield slightly different results but that the overall percentage identity of two sequences is not significantly altered when using different algorithms.

A "mature polypeptide" is defined herein as a polypeptide in its final form and is obtained after translation of a mRNA into a polypeptide and post-translational modifications of said polypeptide. Post -translational modifications include N-terminal processing, C-terminal truncation, glycosylation, phosphorylation and removal of leader sequences such as signal peptides, propeptides and/or prepropeptides by cleavage.

A mature polypeptide sequence of SEQ ID NO: 1 may comprise or contain amino acids 19, 20, 21, 22, 23, 24 to 640 of the amino acid sequence of SEQ ID NO: 1, advantageously the mature polypeptide sequence of SEQ ID NO: 1 comprises or contains amino acids 22 to 640 of SEQ ID NO: 1, wherein methionine at position 1 in SEQ ID NO: 1 is counted as number 1.

The term "polypeptide" refers to a molecule comprising amino acid residues linked by peptide bonds and containing more than five amino acid residues. The term "protein" as used herein is synonymous with the term "polypeptide" and may also refer to two or more polypeptides. Thus, the terms "protein" and "polypeptide" can be used interchangeably. Polypeptides may optionally be modified (e.g., glycosylated, phosphorylated, acylated, farnesylated, prenylated, sulfonated, and the like) to add functionality. Polypeptides exhibiting activity in the presence of a specific substrate under certain conditions may be referred to as enzymes.

A peptidyl arginine deiminase, or polypeptide having peptidyl arginine deiminase activity may be produced in any suitable host organism by known methods in the art, for instance in fungi *Aspergilli, eg Aspergillus niger* or *Aspergillus oryzae, Trichoderma,* or the yeasts *Saccharomyces,* and *Kluyveromuces* or the bacteria of the genus *Streptomyces* or *Bacilli.* A suitable method to express a polypeptide having peptidyl arginine deiminase activity in *Aspergillus niger* is for instance disclosed in Examples 3 and 4 in WO2008/000714.

In one embodiment a use of peptidyl arginine deiminase according to claim 1 to improve a physical property of a protein, further comprises the use of a protein glutaminase.

In one embodiment a process of improving a physical property according to claim 3 comprises further incubating the protein with a protein glutaminase.

Surprisingly, it was found that when a use according to claim 1 further comprises the use of a protein glutaminase or a process according to claim 3 for improving a solubility further comprises incubating protein with a protein glutaminase a higher amount of protein was solubilized than when the protein was incubated or solubilized with either peptidyl arginine deiminase or protein glutaminase alone.

A protein glutaminase is an enzyme that hydrolyzes amid groups of glutamine and / or asparagine in a protein to glutamic acid and / or asparaginic acid and ammonia. A protein glutaminase belongs to enzyme classification EC 3.5.1.44.

A protein glutaminase may be derived from any suitable microorganism, for instance bacteria from the genus *Chryseobacterium, Flavobacterium, Empedobacter, Sphingobacterium, Bacillus, Aureobacterium* or *Myroides* for instance *Chryseobacterium gleum, C, indologenes, C. meningosepticum C. proteolyticum, Flavobacterium aquatile, Empedobacter brevis, Sphingobacterium spiritivorum, S. heparinum, Bacillus circulans Aureobacterium esteroaromaticum* or *Myroides odoratus.* A protein glutaminase may be derived from *C. proteolyticum.* For instance a protein glutaminase is a polypeptide which has at least 80%, 85%, 90%, 95%, 96%, 97%, 98%, 99% or 100% identity to the amino acid sequence of SEQ ID NO: 2, or to the mature amino acid sequence of SEQ ID NO: 2, wherein the polypeptide has protein glutaminase activity.

A mature polypeptide sequence of SEQ ID NO: 2 may comprise or contain amino acids 136 to 320 of SEQ ID NO: 2, wherein methionine at position 1 in SEQ ID NO: 2 is counted as number 1.

A protein glutaminase may be produced in any suitable organism by methods known to a person skilled in the art. A protein glutaminase may be produced in bacteria for instance from the genus *Pseudomonas, Bacillus* or *Escherichia,* for instance *Pseudomonas putida, Bacillus subtilis* or *Escherichia coli.*

A protein glutaminase may be a pure or purified protein glutaminase. A pure of purified protein glutaminase is an enzyme that may be at least 50% pure, e.g., at least 60% pure, at least 70% pure, at least 75% pure, at least 80% pure, at least 85% pure, at least 80% pure, at least 90% pure, or at least 95% pure, 96%, 97%, 98%, 99%, 99.5%, 99.9% pure for instance as determined by SDS-PAGE or any other analytical method suitable for this purpose and known to the person skilled in the art.

In one embodiment the present disclosure relates to a process according to claim 3 to improve solubility of a plant protein chosen from pea, soy or cereal and optionally to reduce foam capacity of said plant protein comprising incubating the protein with a peptidyl arginine deiminase, wherein the protein is solubilized and optionally foam capacity is reduced. The solubility of the protein is increased and optionally the foam capacity of the protein is reduced compared to a protein that has not been incubated with a protein arginine deiminase in a process as disclosed herein.

The protein may be incubated with peptidyl arginine deiminase and optionally protein glutaminase at any suitable pH and temperature. Preferably, the protein is incubated with peptidyl arginine deiminase and optionally protein glutaminase at a pH of between 5 and 8.5, such as a pH of between 5.5 and 8, such as a pH of between 6 and 7, or a pH of between 6.2 and 6.8. A suitable temperature at which protein is incubated with peptidyl arginine deiminase and optionally protein glutaminase may be between 20 and 60 degrees Celsius, such as a between 30 and 50, or between 35 and 45 degrees Celsius.

Plant protein as used herein may be a solution comprising plant protein selected from pea, soy or cereal protein. A solution comprising plant protein may be a beverage comprising plant protein, for instance a beverage may be milk derived derived from plant sources. Plant sources of milk include, but are not limited to, milk extracted from soy bean, pea, peanut, barley, rice, oat, quinoa, almond, cashew, coconut, hazelnut, hemp, sesame seed and sunflower seed .

## FIGURES

**Figure 1.** SDS PAGE analysis of various proteins incubated with and without PAD. Lane 1: MW marker; Lane 2: pea protein; Lane 3: pea protein +PAD; Lane 4: wheat gluten; Lane 5: wheat gluten +PADLane; 6: soy protein; Lane 7: soy protein + PADLane; 8: Bone collagen; Lane 9: bone collagen +PAD.

**Figure 2.** The solubility of pea protein at different pH values incubated with PAD at pH 6.5 and at 40°C.

**Figure 3.** Foam capacity of pea protein treated with PAD and PAD and PG at pH 6.5 and at 40°C compared to the foam capacity of untreated pea protein and egg white

## EXAMPLES

## MATERIALS

### Molecular biology techniques

Molecular biology techniques known to the skilled person are performed as set out in Sambrook & Russell, Molecular Cloning: A Laboratory Manual, 3rd Ed., CSHL Press, Cold Spring Harbor, NY, 2001).

### Cloning and expression of peptidyl arginine deiminase in *Aspergillus niger*

Cloning and expression of the polypeptide having peptidyl arginine demininase activity according to SEQ ID NO: 1, was performed as disclosed in Examples 3 and 4 of WO2008/000714.

### Peptidyl arginine deiminase activity

Peptidylarginine deiminase activity was determined by measuring the formation of citrulline residues in α-N-Benzoyl-L-arginine-ethyl ester (BAEE). The incubation mixture contained 100mM tris-HCl buffer (pH 7.5), 5 mM CaCl2, 10 mM DTT, 10 mM BAEE in a final volume of 700 μl. Incubation was performed at 55°C for 30 min, and the reaction was stopped by adding 100μl 8 N $HClO_4$. Citrulline was determined by colorimetry according the method of Guthöhrlein and Knappe, (1968) Anal. Biochem. 26, 188.

One unit of peptidyl arginine deiminase is expressed as 1 μmol of citrulline formed / min / mg of protein.

### Cloning and expression of protein glutaminase (PrgA) of *Chryseobacterium proteolyticum* in *Bacillus subitilis*

The *E. coli B. subtilis* shuttle vector pGBB09 is described in US 8426182 B1, was used for the expression of protein-glutaminase (PrgA) (SEQ ID NO: 2) from *Chryseobacterium proteolyticum* strain 9670 (S. Yamaguchi, David J. Jeenes and David B. Archer (2001) Eur. J. Biochem. 268, 1410-1421). The *prgA* gene was synthesised with a PacI restriction site and ribosome binding site at the 5'- end and a double stop codon and *Pme*I restriction site at the 3'end this DNA fragment is listed as SEQ ID NO: 3. The fragment was cloned into vector pGBB09 using the *Pac*I and *Pme*I restriction sites and this results in the PrgA expression vector pBHA12-PGU-1. The sequence of the plasmid was confirmed by DNA sequencing.

The vector pBHA12-PGU-1 was transformed into *B. subtilis* strain BS154 (CBS 363.94) (Δ*aprE*, Δ*nprE, amyE, spo*-) as described in Quax and Broekhuizen 1994 Appl Microbiol Biotechnol. 41: 425-431. The *B. subtilis* strain BS154 containing the PrgA expression vector was named BSU154PGU-1. *B. subtilis* BSU154 PGU-1 was grown in a shake flask. These shake flasks contained 20 ml 2xTY medium composed of 1.6% (w/v) Bacto tryptone, 1% (w/v) Yeast extract and 0.5% (w/v) NaCl. The cultures were shaken vigorously at 37°C and 250 rpm for 16 hours and 0.2 ml culture medium was used to inoculate 20 ml SMM medium.

SMM pre-medium contained 1.25% (w/w) yeast extract, 0.05% (w/w) $CaCl_2$, 0.075% (w/w) $MgCl_2.6H_2O$, 15 μg/l $MnSO_4$ .$4H_2O$, 10 μg/l $CoCl_2.6H_2O$, 0.05% (w/w) citric acid, 0.025% (w/w) antifoam 86/013 (Basildon Chemicals, Abingdon, UK). To complete SMM medium, 20 ml of 5% (w/v) maltose and 20 ml of a 200 mM Na-phosphate buffer stock solution (pH 6.8), both prepared and sterilized separately, were added to 60 ml SMM pre-medium.

The cultures were incubated at 37°C and 250 rpm for 48 hours. The supernatants were harvested by centrifugation at 13000 rpm5 minutes for 5 minutes after which they were stored at -20 °C until further use.

**Protein glutaminase (PG) activity**

The activity of PG was measured using a modified method described by Yamaguchi S. et al in Appl. Environ. Microbiol. (2000) Vol 66, No. 8, p 3337-3343 . 10mM Z-Gln-Gly (Sigma) substrate was prepared in 65mM sodium phosphate buffer at pH 6.5 and incubated with the enzyme at 40°C. The reaction was stopped with 255 trichloroacetic acid followed by centrifugation at 20000g for 5min. The ammonia released was measured by the Berthelot method. The enzyme activity was expressed as the amount of ammonia (in mmol) released at 40°C in 30 min.

**Example 1. Peptidyl arginine deiminase (PAD) is active on various protein substrates**

Peptidyl arginine deiminase (PAD) converts arginine to citrulline whereby ammonia is released. Therefore, the ammonia release is a measure of enzyme activity on the chosen protein substrates.

40 mg protein substrates from soy protein isolate (Wilmar International Ltd), pea protein isolate (Pisane F9, Cosucra), wheat gluten (Sigma) and insoluble collagen (Sigma) were weighed into 2 ml Eppendorf cups and 1 ml of 50 mM sodium phosphate pH 6.5 was added. The incubations were started by adding PAD enzyme at a dose of 0.1% v/w on dry matter. After 16 hours incubation at 40°C the samples were centrifuged for 10 minutes at 14000 rpm at 25-40°C. The samples were diluted once, except for the collagen, which was diluted twice, and samples were centrifuged again over the 10K PES filter. Protein substrates without PAD addition were incubated under the same conditions to serve as a Blanc. The supernatant of the samples were stored at 4°C.

Ammonia analysis was performed with Ammonia Assay Kit (Sigma -Aldrich).

The kit is designed for quantitative, enzymatic determination of ammonia in food and biological samples. Ammonia reacts with α-ketoglutaric acid and reduced NADPH in the presence of $_L$-glutamate dehydrogenase to form $_L$-glutamate and $NADP^+$. The decrease in absorbance at 340 nm (NADPH → $NADP^+$) is proportional to the ammonia concentration.

100 μl of the supernatant from the protein incubations were added to 1 ml Ammonia assay Reagent. After mixing the solution was incubated for 5 minutes at room temperature and the absorbance at 340 nm was determined. Subsequently 10 μl of the Dehydrogenase was added. The absorbance was measured again after 5 minutes incubation. The amount of ammonia was calculated using the Molar Extinction Coefficient of NADPH. An ammonia standard solution was used as a control.

Table 1 shows the amount of ammonia released after incubating the protein substrate with PAD at 40°C for 16 h, minus the amount of ammonia measured in the substrate incubated without PAD.

**Table 1.** The ammonia released from various protein substrates after incubation with PAD at 40°C for 16 h

| Substrate | Amount of ammonia released (μg $NH_3$/ml) |
|---|---|
| soy | 29.5 |
| pea | 22.1 |
| gluten | 25.0 |
| collagen | 25.4 |

**Example 2. Changes in the protein solubilization after incubation with peptidyl arginine deiminase**

Soy protein isolate (Wilmar International Ltd) pea (Pisane C9, Cosucra), wheat gluten (Sigma) and insoluble collagen (Sigma) protein substrates were incubated with peptidyl arginine deiminase (PAD) and the amount of protein solubilized was measured.

200 mg substrate was mixed with 4.8 ml 50 mM sodium phosphate buffer pH 6.5 in Greiner tubes containing a magnetic stirrer and placed in a water-bath with a device for magnetic stirring of 40°C. 0.1 % PAD enzyme protein on dry matter was added. The samples were incubated overnight and centrifuged for 25 minutes at 3220*g. The supernatants were again centrifuged for 20 minutes at 14000 rpm.

The samples were used to measure protein solubilization by measuring the nitrogen (N) content in the supernatant after centrifugation using Kjeldahl analysis. The results of the protein analysis is presented in Table 2.

**Table 2.** Protein solubilization after incubation with PAD at 40°C for 16 h, measured as nitrogen (Kjeldahl)

| Substrate protein | Kjeldahl nitrogen (mg/kg) | | % increase in the solubilization by PAD at pH 6.5 |
|---|---|---|---|
| | PAD addition | | |
| | - | + | |
| soy | 656 | 898 | 37 |
| pea | 493 | 734 | 49 |
| gluten | 327 | 639 | 95 |
| collagen | 5320 | 5510 | 3.5 |

**SDSPAGE analysis**

The protein substrates incubated with peptidyl arginine deminase were analyzed by SDS-PAGE.

65 µl of the supernatants was mixed with 25 µl LiDS sample buffer and 10 µl sample reducing agent. The mixture was heated for 15 minutes at 70°C. Subsequently 10 µl of each sample was applied to the 10% Bis-Tris gel and proteins were separated using MES buffer as running system for 39 minutes at 200V. The gel was stained with Instant Blue.

The SDS page gel shown in Figure 1 shows that no proteolysis was observed in the incubations of the different protein substrates with peptidyl arginine deiminase.

**Example 3: Solubility of plant proteins treated with peptidyl arginine deiminase or a**

**combination of peptidyl arginine deminase and protein glutaminase**

A dispersion of 100mg/ml of plant proteins from pea protein (Pisane C9, Cosucra), soy protein isolate (Wilmar Int. Ltd.) and rice protein isolate was prepared in water at pH 6.5. To these dispersions, 0.1 wt/wt % PAD or wt/wt 0.1% PAD and 0.1 wt/wt % PG enzyme protein to dry matter was added and incubatedin a thermostat mixer for 4h at 40°C. After 4 h the material was centrifuged at 5600g for 10min. The percent solubilization for each protein material was calculated from the protein content in the supernatant after centrifugation.

The protein quantification was performed using the Biuret method after thricloroacetic acid precipitation of the proteins.

Table 3 shows that a higher amount of protein is solubilized when the protein was incubated with both PAD and PG than the amount of protein solubilized after incubation with either PAD or PG alone.

**Table 3.** Solubilization of pea, soy and rice protein after incubation with PAD, PG and a combination of PAD+PG at pH 6.5 and 40°C during 4 h

| **Protein substrate** | **% Solubilized protein at pH 6.5** | | | |
|---|---|---|---|---|
| | **control** | **PAD** | **PG** | **PAD+PG** |
| **Pea** | 20 | 31 | 36 | 43 |
| **Soy** | 12 | 14 | 18 | 21 |
| **Rice** | 0.8 | 1 | 1.3 | 1.5 |

**Example 4. pH dependence of the solubility of pea proteins treated with peptidyl arginine deiminase**

A 15 wt/wt % pea protein material was treated with 0.1 % w/w PAD enzyme to protein at pH 6.5 and 40°C for 16h. The enzyme was inactivated by heat shock at 85°C for 15 min and the protein material was then freeze dried. A 1 wt/wt % solution of enzyme treated pea protein as described above was prepared in water at different pH values ranging from pH 8 to pH 3.

The pH was adjusted in steps up to pH 8 with addition of 4N sodium hydroxide and then down up to pH 3 by addition of 4N sulfuric acid. At each step of pH adjustment an aliquot was taken, mixed for another 2h at room temperature and then centrifuged for 5 minutes at 20,000 g. The protein in the supernatant was then precipitated with trichloroacetic acid and the protein quantified by the Biuret method. Figure 2 shows that at a pH of between 5 and 8 peptidyl arginine deiminase was able to increase pea protein solubility with about 2 to 10%.

**Example 5. Foam capacity of a solution containing pea protein treated with peptidyl arginine deiminase and a combination of peptidyl arginine deiminase and protein glutaminase**

Pea protein, (Pisane C9, Cosucra) was suspended in water at 15 wt/wt% Dry Matter and incubated at pH 6.5 and at 40°C for 16h, with enzymes (0.1% v/w) PAD, PG or PAD and PG as described in the Example 4. The protein slurries were freeze dried and the protein content in the dry matter was measured by Kjeldahl analysis.

Foaming experiments were performed in 3 wt/wt% protein solution of pea protein treated with enzymes. The foaming experiments were performed at a 10 ml scale at 22°C and pH 6.5.

The 10 ml 3 wt/wt% pea protein solution was mixed with a Multimixer in a 50 ml measuring cylinder during 60 seconds. Foam volume was measured after 0 and 30 minutes. The foam capacity was calculated as follows:

$$\text{Foam capacity (FC)} = (\text{Foam volume at T=0/start liquid volume}) \text{ x100\%}.$$

Figure 3 shows that the foam capacity of the pea protein treated with PAD, PG and PAD and PG was reduced compared to the foam capacity of untreated pea protein.

## Claims

1. Use of peptidyl arginine deiminase (EC 3.5.3.15) to improve solubility of a plant protein at a pH of between 5 and 8.5, wherein the plant protein is pea, soy or cereal.

2. Use according to claim 1, further comprising the use of a protein glutaminase (EC 3.5.1.44).

3. A process to improve solubility of a plant protein comprising incubating the plant protein with a peptidyl arginine deiminase at a pH of between 4 and 9, wherein the plant protein shows an improved solubility at a pH of between 5 and 8.5, wherein the plant protein is pea, soy or cereal protein.

4. A process according to claim 3, wherein the plant protein is further incubated with a protein glutaminase (EC 3.5.1.44).

5. Use according to claim 1 or 2, or process according to claim 3 or 4, wherein the use or the process further comprises reducing foam capacity of the protein.

6. Use according to claims 1, 2 or 5, or a process according to any one of the claims 3 to 5, wherein the solubility of the protein is increased with at least 2%.

7. Use according to any one of the claims 1, 2, 5 or 6 or a process according to any one of the claims 3 to 6, wherein the cereal protein is gluten.

8. Use according to any one of the claims 1, 2, 5 or 6 or 7 or a process according to any one of the claims 3 to 7, wherein the plant protein is a solution comprising plant protein.

9. Use or process according to claim 8, wherein the solution is a beverage.

10. Use or process according to claim 9, wherein the beverage is milk derived from a plant source.

11. Use according to any one of the claims 1, 2, 5 to 10, or a process according to any one of the claims 3 to 10, wherein the peptidyl arginine deiminase has at least 80% identity to SEQ ID NO: 1, or to the mature amino acid sequence of SEQ ID NO: 1.

## Patentansprüche

1. Verwendung von Peptidylarginindeiminase (EC 3.5.3.15) zum Verbessern der Löslichkeit eines Pflanzenproteins bei einem pH-Wert zwischen 5 und 8,5, wobei es sich bei dem Pflanzenprotein um Erbse, Soja oder Getreide handelt.

2. Verwendung nach Anspruch 1, weiterhin umfassend die Verwendung einer Proteinglutaminase (EC 3.5.1.44).

3. Verfahren zur Verbesserung der Löslichkeit eines Pflanzenproteins, bei dem man das Pflanzenprotein bei einem pH-Wert zwischen 4 und 9 mit einer Peptidylarginindeiminase inkubiert, wobei das Pflanzenprotein eine verbesserte Löslichkeit bei einem pH-Wert zwischen 5 und 8,5 zeigt, wobei es sich bei dem Pflanzenprotein um Erbsen-, Soja- oder Getreideprotein handelt.

4. Verfahren nach Anspruch 3, wobei das Pflanzenprotein weiterhin mit einer Proteinglutaminase (EC 3.5.1.44) inkubiert wird.

5. Verwendung nach Anspruch 1 oder 2 oder Verfahren nach Anspruch 3 oder 4, wobei die Verwendung bzw. das Verfahren weiterhin die Verminderung der Schäumungskapazität des Proteins umfasst.

6. Verwendung nach Ansprüchen 1, 2 oder 5 oder Verfahren nach einem der Ansprüche 3 bis 5, wobei die Löslichkeit des Proteins um mindestens 2% erhöht wird.

7. Verwendung nach einem der Ansprüche 1, 2, 5 oder 6 oder Verfahren nach einem der Ansprüche 3 bis 6, wobei es sich bei dem Getreideprotein um Gluten handelt.

8. Verwendung nach einem der Ansprüche 1, 2, 5 oder 6 oder 7 oder Verfahren nach einem der Ansprüche 3 bis 7, wobei es sich bei dem Pflanzenprotein um eine Pflanzenprotein umfassende Lösung handelt.

9. Verwendung oder Verfahren nach Anspruch 8, wobei es sich bei der Lösung um ein Getränk handelt.

10. Verwendung oder Verfahren nach Anspruch 9, wobei es sich bei dem Getränk um Milch pflanzlichen Ursprungs handelt.

11. Verwendung nach einem der Ansprüche 1, 2, 5 bis 10 oder Verfahren nach einem der Ansprüche 3 bis 10, wobei die Peptidylarginindeiminase mindestens 80% Identität mit SEQ ID NO: 1 oder mit der reifen Aminosäuresequenz von SEQ ID NO: 1 aufweist.

## Revendications

1. Utilisation d’une peptidylarginine déiminase (EC 3.5.3.15) pour améliorer la solubilité d’une protéine végétale à un pH compris entre 5 et 8, 5, la protéine végétale étant de pois, de soja ou de céréale.

2. Utilisation selon la revendication 1, comprenant en outre l’utilisation d’une protéine glutaminase (EC 3.5.1.44).

3. Procédé pour améliorer la solubilité d’une protéine végétale comprenant l’incubation de la protéine végétale avec une peptidylarginine déiminase à un pH compris entre 4 et 9, la protéine végétale présentant une solubilité améliorée et à un pH compris entre 5 et 8,5, la protéine végétale étant une protéine de pois, de soja ou de céréale.

**4.** Procédé selon la revendication 3, la protéine végétale étant en outre incubée avec une protéine glutaminase (EC 3.5.1.44).

**5.** Utilisation selon la revendication 1 ou 2, ou procédé selon la revendication 3 ou 4, l'utilisation ou le procédé comprenant en outre la réduction de la capacité moussante de la protéine.

**6.** Utilisation selon les revendications 1, 2 ou 5, ou procédé selon l'une quelconque des revendications 3 à 5, la solubilité de la protéine étant augmentée d'au moins 2 %.

**7.** Utilisation selon l'une quelconque des revendications 1, 2, 5 ou 6 ou procédé selon l'une quelconque des revendications 3 à 6, la protéine de céréale étant le gluten.

**8.** Utilisation selon l'une quelconque des revendications 1, 2, 5 ou 6 ou 7 ou procédé selon l'une quelconque des revendications 3 à 7, la protéine végétale étant une solution comprenant une protéine végétale.

**9.** Utilisation ou procédé selon la revendication 8, la solution étant une boisson.

**10.** Utilisation ou procédé selon la revendication 9, la boisson étant un lait issu d'une source végétale.

**11.** Utilisation selon l'une quelconque des revendications 1, 2, 5 à 10, ou procédé selon l'une quelconque des revendications 3 à 10, la peptidylarginine déiminase possédant au moins 80 % d'identité à la SEQ ID n° : 1, ou à la séquence d'acides aminés mature de SEQ ID n° : 1.

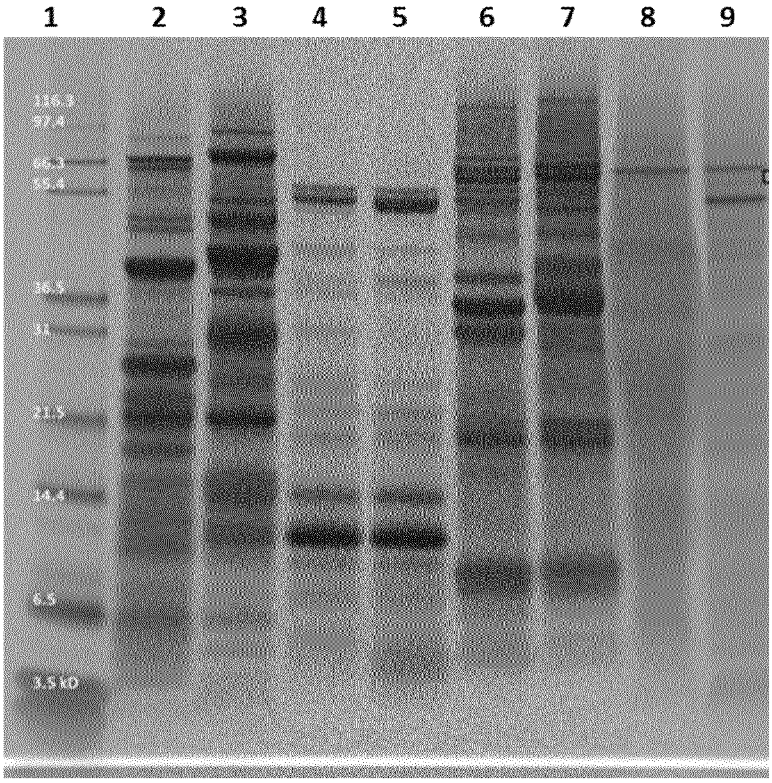


Fig.1

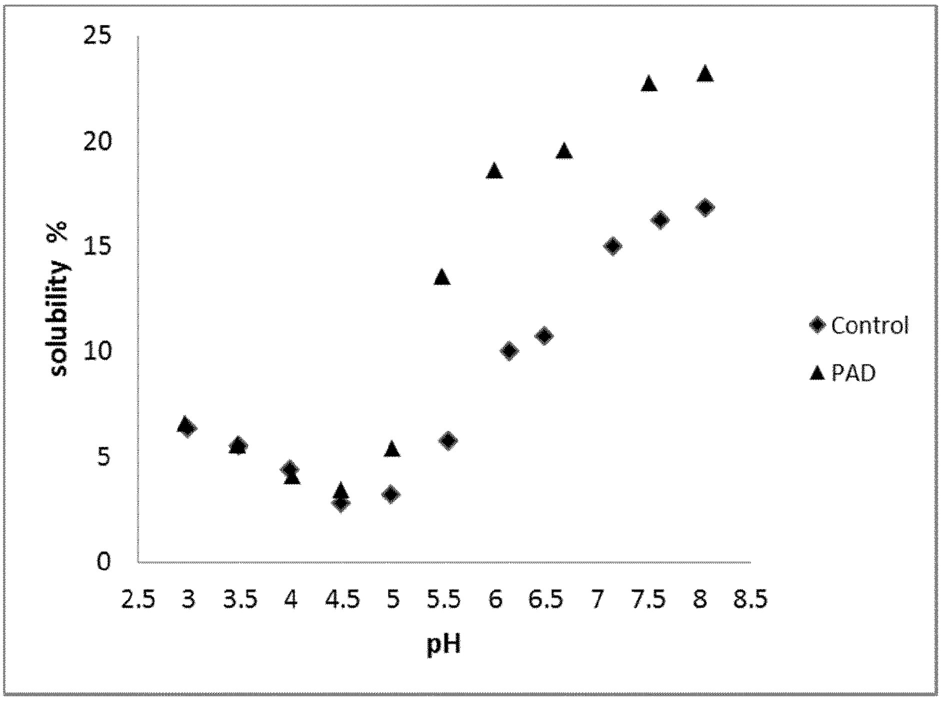
25
20
15
10
5
0
solubility %
2.5
3
3.5
4
4.5
5
5.5
6
6.5
7
7.5
8
8.5
pH
Control
PAD

Fig. 2

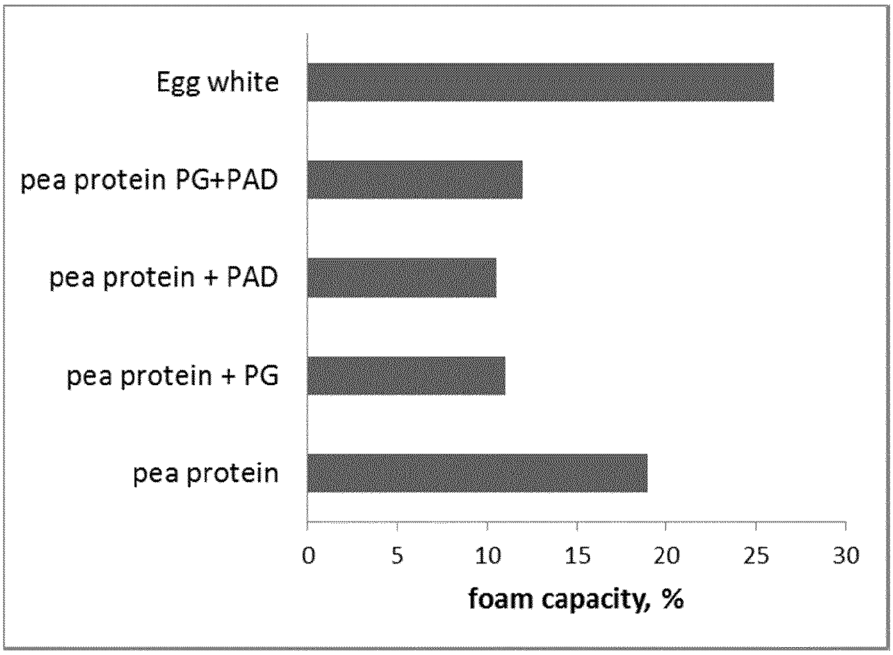
Egg white
pea protein PG+PAD
pea protein + PAD
pea protein + PG
pea protein
0
5
10
15
20
25
30
foam capacity, %

Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008000174 A
- WO 2008000714 A
- US 8426182 B1


### Non-patent literature cited in the description


- **YONG et al.** *J. of Agric. Food Chem.,* 2006, vol. 54, 6034-6040
- **NEEDLEMAN, S. B. ; WUNSCH, C. D.** *J. Mol. Biol.,* 1970, vol. 48, 443-453
- **RICE,P ; LONGDEN, I ; BLEASBY, A.** EMBOSS: The European Molecular Biology Open Software Suite. *Trends in Genetics,* 2000, vol. 16 (6), 276-277
- **SAMBROOK ; RUSSELL.** Molecular Cloning: A Laboratory Manual. CSHL Press, 2001
- **GUTHÖHRLEIN ; KNAPPE.** *Anal. Biochem.,* 1968, vol. 26, 188
- **S. YAMAGUCHI ; DAVID J. JEENES ; DAVID B. ARCHER.** *Eur. J. Biochem.,* 2001, vol. 268, 1410-1421
- **QUAX ; BROEKHUIZEN.** *Appl Microbiol Biotechnol.,* 1994, vol. 41, 425-431
- **YAMAGUCHI S. et al.** *Appl. Environ. Microbiol.,* 2000, vol. 66 (8), 3337-3343